Europäisches Patentamt

European Patent Office

Office européen des brevets

(11) Numéro de publication: **0 437 393 B1**

# FASCICULE DE BREVET EUROPEEN

(45) Date de publication du fascicule du brevet: **08.11.95**

(51) Int. Cl.⁶: **C12N 9/02**, C12M 1/00, C12P 1/00, C12N 1/12

(21) Numéro de dépôt: **91400032.8**

(22) Date de dépôt: **09.01.91**

(54) **Procédé de production et d'extraction d'anti-oxydants à partir d'une culture de microorganismes et photobioréacteur pour la mise en oeuvre de ce procédé.**

(30) Priorité: **11.01.90 FR 9000279**

(43) Date de publication de la demande:
**17.07.91 Bulletin 91/29**

(45) Mention de la délivrance du brevet:
**08.11.95 Bulletin 95/45**

(84) Etats contractants désignés:
**BE CH DE ES GB IT LI NL**

(56) Documents cités:
**EP-A- 0 310 522**

**CHEMICAL ABSTRACTS, vol. 87, 1977, Columbus, OH (US); H.P. MISRA et al., p. 213, no. 147729z**

**CHEMICAL ABSTRACTS, vol. 74, 1971, Columbus, OH (US); N.J. ANTIA et al., p. 99, no. 29131g**

(73) Titulaire: **HELIOSYNTHESE S.A.**
**Centre d'Affaires Actimart**
**Bureau 218**
**1140 rue Ampère**
**F-13795 Aix en Provence Cédex (FR)**

(72) Inventeur: **Gudin, Claude**
**4 Traverse Sainte Anne,**
**Le Parc**
**F-13100 Aix en Provence (FR)**
Inventeur: **Thepenier, Catherine**
**Les Restanques de la Thomassine**
**F-04100 Manosque (FR)**

(74) Mandataire: **Gandrille, Pierre et al**
**Société Brevatome**
**Sté de Protection des Inventions**
**25, rue de Ponthieu**
**F-75008 Paris (FR)**

EP 0 437 393 B1

# Description

La présente invention a pour objet un procédé de production et d'extraction d'anti-oxydants à partir d'une culture de micro-organismes photosynthétiques en suspension dans un milieu liquide ainsi que le photobioréacteur pour la culture de ces micro-organismes. Ce procédé permet une production intensive et contrôlée d'anti-oxydants à l'échelle industrielle.

L'invention s'applique à la production de tout anti-oxydant et en particulier à la production des enzymes superoxydes-dismutases, connues sous l'abréviation SOD, de la vitamine C, des tocophérols alpha, béta ou gamma, l'alpha-tocophérol étant la vitamine E.

Les superoxydes-dismutases sont des métalo-enzymes comportant deux sous-unités peptidiques liées entre elles. On connaît trois types de SOD classés en fonction de la nature du métal :

- les SOD à Cu/Zn qui sont fréquentes chez les eucaryotes; la source traditionnelle de ces SOD est le sang de boeuf ; elles sont extraites des érythrocytes;
- les SOD à Mn que l'on trouve chez les eucaryotes au niveau des mitochondries et chez les procaryotes;
- enfin, les SOD à Fe qui semblent spécifiques des procaryotes, c'est-à-dire des bactéries aérobies.

Les SOD sont des catalyseurs de la dismutation des ions superoxydes suivant la réaction :

$$O_2^- + O_2^- + 2 H^+ \longrightarrow H_2O_2 + O_2$$

Aussi, les SOD sont utilisées dans les phénomènes aigus libérant des radicaux superoxydes ou dans les phénomènes de vieillissement. Les SOD sont destinées en particulier aux domaines pharmaceutique comme anti-inflammatoire (rhumatisme, arthrite), cosmétologique pour la protection de la peau et des cheveux et par exemple comme protecteur d'ultraviolets, agro-alimentaire (comme protecteur des huiles et corps gras, comme agent de régulation de la coloration ou comme engrais) ainsi que dans le domaine bio-médical.

L'utilisation des SOD en cosmétologie est notamment décrite dans le document FR-A-2 297 899.

L'invention s'applique à tout micro-organisme photosynthétique du type micro-algues et du type cyanobactéries. L'une des cyanobactéries la plus connue est "l'algue bleue".

La photosynthèse est la transformation, grâce à l'énergie solaire, du dioxyde de carbone en matière première hydrocarbonée, l'oxygène étant le principal sous-produit de cette transformation biochimique. Cette réaction peut être symbolisée par l'équation de Myers suivante:

$$6,14 \ CO_2 + 3,65 \ H_2O + NH_3 \longrightarrow C_{6,14}H_{10,3}O_{2,24}N + 6,85 \ O_2$$

Le terme $C_{6,14}H_{10,3}O_{2,24}N$ correspond aux micro-organismes, appelés encore biomasse.

Dans le document FR-A-2 621 323 déposé le 2 octobre 1987 au nom du demandeur, il est décrit un dispositif de production intensive et contrôlée de micro-organismes photosynthétiques. Ce dispositif est en particulier un photobioréacteur clos conçu de façon à contrôler la monoculture d'une espèce désirée ainsi que les paramètres de la culture, comme la température, le pH, les pressions de $CO_2$ et de $O_2$ dans le milieu de culture, ainsi que a composition du milieu nourricier afin de favoriser la production de l'espèce choisie.

Ces photobioréacteurs sont constitués essentiellement d'un récepteur ou capteur solaire transparent à la lumière dans lequel circule le milieu liquide contenant les micro-organismes, un carbonateur raccordé à l'entrée du capteur solaire pour charger le milieu liquide en $CO_2$ nécessaire à la photosynthèse ainsi qu'un dégazeur raccordé au photobioréacteur pour éliminer du milieu liquide l'oxygène produit par les micro-organismes ainsi que le $CO_2$ non dissous dans le milieu liquide.

Le carbonateur permet d'assurer un transfert suffisant de $CO_2$ de la phase gazeuse à la phase liquide afin que la demande biologique en $CO_2$ soit toujours satisfaite. Le $CO_2$ n'est alors pas un facteur limitant la production de micro-organismes.

Si les autres conditions de culture sont favorables, telles que la lumière, le milieu nourricier, le pH, etc. il y a une forte demande en $CO_2$, ce qui entraîne un fort dégagement d'oxygène. Aussi, dans la partie récepteur solaire du photobioréacteur, il y a un enrichissement de la culture en $O_2$ dissous ainsi que des poches gazeuses d'$O_2$.

Dans le document FR-A-2 621 323, on enseigne l'élimination grâce à un dégazeur de l'oxygène produit par photosynthèse, contrariant l'exploitation du photobioréacteur et en particulier la bonne régulation thermique de celui-ci (séchage des micro-organismes, mauvais rendement du photobioréacteur). En outre, l'oxygène peut être toxique pour certains micro-organismes.

Contrairement à l'enseignement de ce document, les inventeurs ont trouve que la collection de l'oxygène produit par photosynthèse et sa réinjection dans le milieu de culture permettaient une production importante d'anti-oxydants par les micro-organismes cultivés. En effet, dans les conditions de forte teneur en oxygène, les micro-organismes photosynthétiques réagissent, face à ces conditions oxydantes, en synthétisant des anti-oxydants.

L'invention a donc pour objet un procédé de production et d'extraction d'anti-oxydants, comportant les étapes suivantes :

(a) - culture dans un photobioréacteur clos de micro-organismes photosynthétiques en suspension dans un milieu liquide de culture, l'oxygène produit par les micro-organismes par photosynthèse étant collecté et réinjecté dans le milieu de culture, ces micro-organismes étant choisis dans le groupe constitué des micro-algues et des cyanobactéries,

(b) - séparation des micro-organismes du milieu de culture,

(c) - mise en solution des micro-organismes séparés en (b),

(d) - "broyage" des micro-organismes en solution,

(e) - addition de solvant à la solution obtenue en (d) pour solubiliser les anti-oxydants produits par les micro-organismes, puis

(f) - séparation des phases liquides et solides en présence.

Avantageusement, l'oxygène est réinjecté sous pression dans le milieu liquide.

Les anti-oxydants produits sont de différents types et sont fonction des espèces cultivées.

Les micro-organismes auxquels s'applique l'invention sont toutes les micro-algues et cyanobactéries connues. Les micro-algues sont en particulier des rhodophycées telles que Porphyridium cruentum qui permet, en condition oxydante, essentiellement la production de SOD du type "Mn".

Cette SOD est constituée de deux sous-unités de 20 000 poids moléculaires chacune qui sont liées par des liaisons non covalentes. Elle présente un point isoélectrique de 4,2 en moyenne.

Porphyridium cruentum produit en moyenne de 10 à 20 U, selon l'analyse par le NBT (nitrobluetétrazolium), par millitre de culture (1 ml de culture contenant environ 1 mg de matière sèche (MS) de micro-algues).

Porphyridium cruentum permet en outre la production de vitamine C. En effet, cette micro-algue contient de 5 à 7g par kg de vitamine C.

Chez cette micro-algue, la voie métabolique "delta-amino-lévulinique" est très développée; elle conduit à des pigments spécifiques (phycobiliprotéines) et à la SOD.

En revanche, la culture de micro-algues du type chlorophycées telles que Haematococcus pluvialis permet en milieu superoxydant essentiellement la production de tocophérols et en particulier de l'alpha-tocophérol (vitamine E) à une concentration de 4 à 5g par kg de micro-algues et du gamma-tocophérol à une concentration de 0,5 à 1g par kg de micro-algues.

Chez cette micro-algue, la voie métabolique "mévalonique" est très développée.

Les conditions favorables de production d'anti-oxydants selon l'invention sont les conditions de forte production d'oxygène, c'est-à-dire de forte activité photosynthétique. Aussi, avantageusement, la culture des micro-organismes est effectuée en condition naturelle d'éclairement et l'extraction des anti-oxydants du milieu de culture et par conséquent l'étape (b) de séparation des micro-organismes du milieu liquide sont effectuées dans l'après-midi.

En outre, les conditions les plus favorables pour la production d'anti-oxydants sont celles où les micro-organismes sont en phase exponentielle de croissance, c'est-à-dire lorsque le taux de croissance est maximal.

Pour un fonctionnement en discontinu ou "batch", la phase exponentielle se trouve en début de culture ; les micro-organismes sont en phase de division active, ce qui correspond à une activité photosynthétique maximale.

En culture continue, il faut choisir un taux de renouvellement rapide du milieu de culture (0,2 à 1 par jour) pour maintenir les micro-organismes en phase de division active.

Avant de séparer les micro-organismes du milieu de culture, on peut effectuer un ou plusieurs traitements de la suspension de micro-organismes ayant pour but d'augmenter la teneur du milieu de culture en anti-oxydants et en particulier en SOD par augmentation de la perméabilité membranaire des cellules.

Ce ou ces traitements peuvent consister en un chauffage de quelques minutes de la suspension cellulaire entre 40 et 50°C ou en une ionisation de cette suspension avec des rayons gamma. Un traitement de 10 kgrays peut entraîner un enrichissement du milieu en activité anti-oxydants de 20 à 100%.

L'étape (b) de séparation des micro-organismes du milieu de culture a pour but de séparer la biomasse, dont on extraira les anti-oxydants intracellulaires au cours des étapes (c) à (f), du milieu de culture qui peut contenir des anti-oxydants exocellulaires.

Dans le cas particulier des SOD, plus de la moitié des enzymes sont dans le milieu de culture et l'activité mesurée dans ce milieu de culture peut, dans certains cas, justifier le traitement du milieu liquide pour en extraire les anti-oxydants exo-cellulaires.

La mise en solution des micro-organismes (étape (c)) permet d'ajuster la concentration du jus des micro-organismes en vue d'améliorer les conditions de "broyage". La concentration optimale des micro-organismes pour l'étape de "broyage" se situe entre 20 et 100 g par litre de matière sèche, selon les espèces utilisées et leur stade de croissance.

L'étape de "broyage" a pour but d'éclater les micro-organismes afin de rendre tout le contenu cellulaire accessible aux solvants. Elle est réalisée dans un homogénéisateur où les micro-organismes subissent une alternance de pressions et de dépressions.

Le solvant ou le mélange de solvants utilisé dans l'étape (e) dépend des anti-oxydants que l'on désire extraire. En particulier, les SOD et la vitamine C sont hydrosolubles et leur extraction se fait en solution aqueuse, alors que les tocophérols sont liposolubles et leur extraction se fait dans un solvant organique du type acétone ou méthanol ou dans une huile végétale ou minérale.

Si l'on désire extraire à la fois les anti-oxydants liposolubles et hydrosolubles produits par une même espèce, on utilise un solvant organique ou une huile.

L'étape (f) de séparation des phases a pour but de séparer le mélange en deux ou trois phases selon que l'on utilise un solvant aqueux ou non : une solution aqueuse contenant les anti-oxydants hydrosolubles, une solution lipidique contenant les anti-oxydants liposolubles et une phase solide constituée des résidus cellulaires. Cette séparation des phases peut être effectuée par centrifugation ou par décantation dans une colonne pulsée.

De préférence, les étapes de "broyage", d'addition de solvant et de séparation (f) des phases sont effectuées à 4°C environ.

En cas de besoin, les phases lipidiques et aqueuses, obtenues au cours de l'étape (f), ainsi que le milieu liquide résultant de l'étape de séparation (b) des micro-organismes peuvent être concentrés et éventuellement purifiés.

Cette concentration peut être réalisée par ultrafiltration et/ou par précipitation avec du sulfate d'ammonium.

La purification est effectuée avantageusement à 4°C et son degré dépend de l'utilisation des anti-oxydants.

L'invention a aussi pour objet un photobioréacteur clos pour la production d'anti-oxydants à partir d'une culture de micro-organismes photosynthétiques en suspension dans un milieu liquide, ces micro-organismes étant choisis dans le groupe constitué des micro-algues et des cyanobactéries, ce photobioréacteur comporte:

(A) - un récepteur solaire transparent à la lumière solaire dans lequel circule le milieu liquide,

(B) - un carbonateur à colonne raccordé à l'entrée du récepteur solaire pour charger le milieu liquide en $CO_2$ nécessaire à la photosynthèse,

(C) - un collecteur de l'oxygène produit par les micro-organismes par photosynthèse, et

(D) - des moyens pour réinjecter l'oxygène collecté au niveau du carbonateur.

En vue d'une activité photosynthétique maximum, le récepteur solaire est constitué de tubes parallèles dans lesquels circule le milieu liquide, des collecteurs d'extrémité assurant la mise en communication de ces tubes.

D'autres caractéristiques et avantages de l'invention ressortiront mieux de la description qui va suivre, donnée à titre illustratif et non limitatif, en référence aux dessins annexés, dans lesquels :

- la figure 1 illustre, schématiquement, les différentes étapes du procédé d'extraction selon l'invention des anti-oxydants à partir d'une culture de micro-algues,

- la figure 2 illustre, schématiquement, un photobioréacteur pour la production de micro-algues conforme à l'invention, fonctionnant en "batch",

- la figure 3 donne l'évolution de l'activité photosynthétique dans le milieu de culture au cours d'une journée, et

- la figure 4 représente schématiquement un photobioréacteur pour la production de micro-algues conformément à l'invention, fonctionnant en continu.

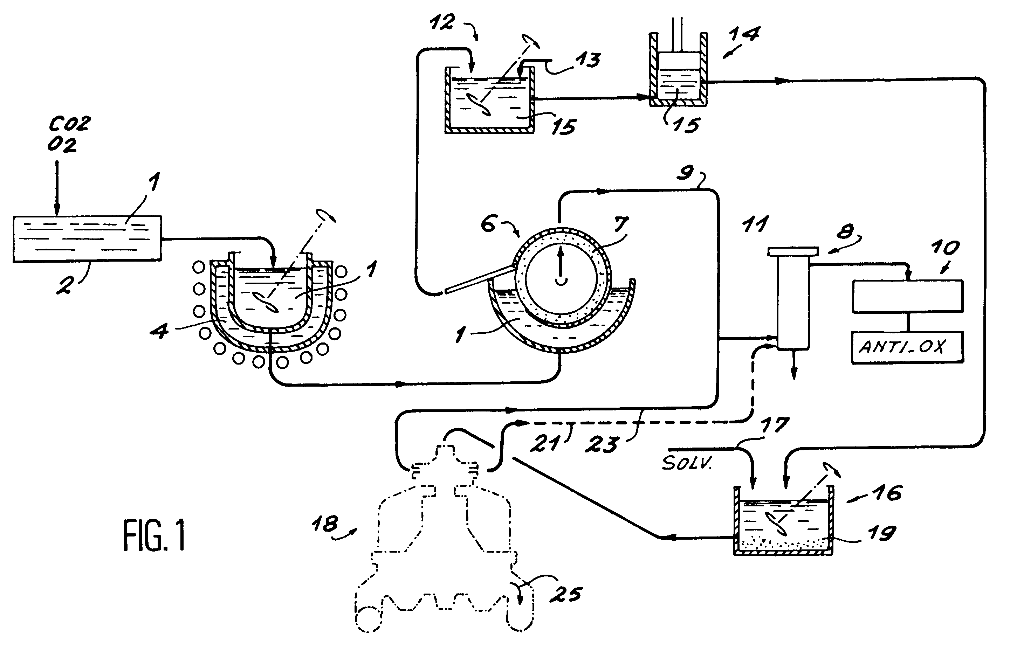

Comme représenté sur la figure 1, la culture 1 de Porphyridium cruentum est réalisée dans un photobioréacteur 2, contenant comme milieu nourricier de l'eau de mer synthétique enrichie en potassium, phosphore et azote. Le potassium est présent sous forme de chlorure, le phosphore sous forme d'acide orthophosphorique et l'azote sous forme d'urée ou de nitrates.

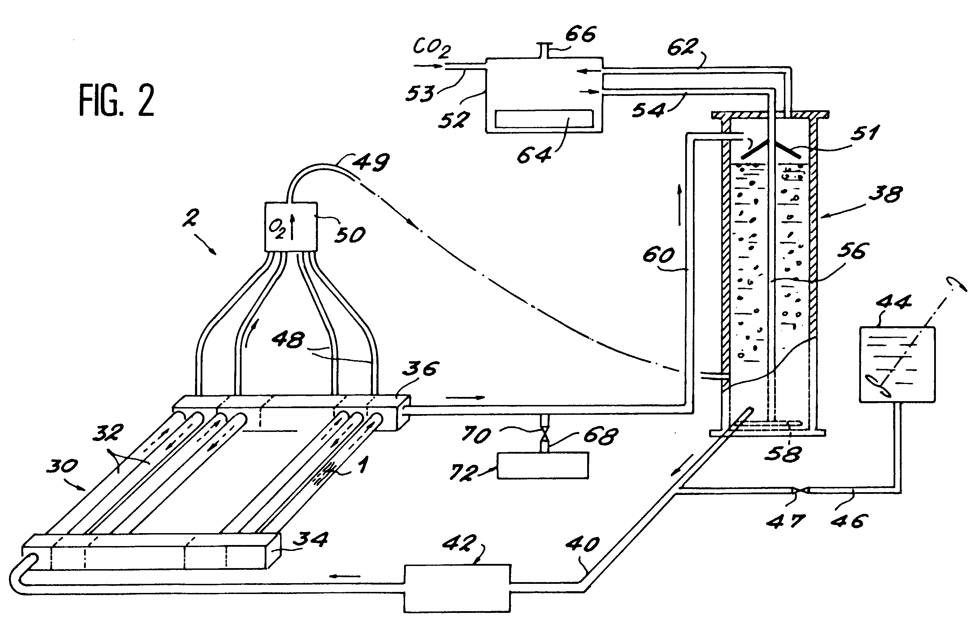

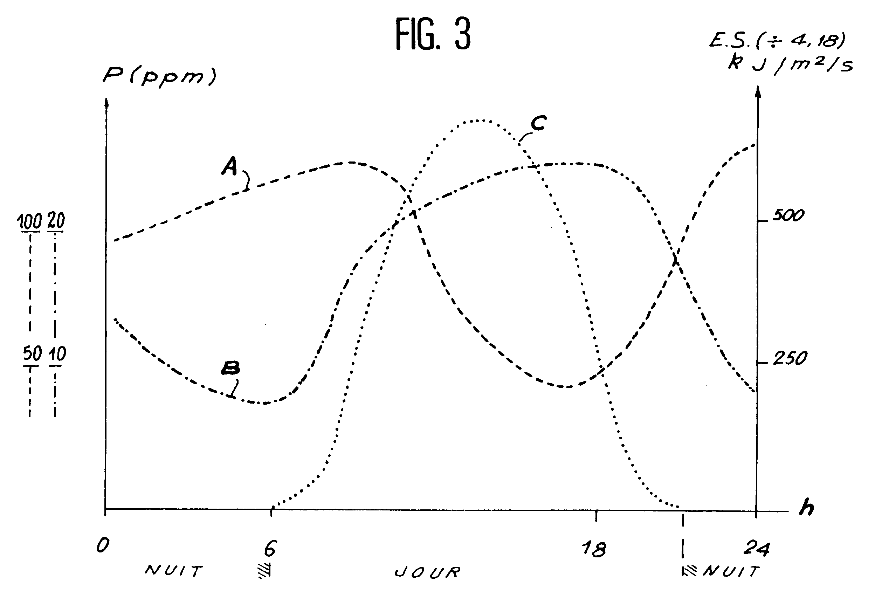

Le pH du milieu de culture est régulé entre 6 et 8 pendant toute la croissance des micro-algues et l'apport en carbone est assuré sous forme de dioxyde de carbone. La réalisation exacte de ce photobioréacteur sera décrite ultérieurement en référence aux figures 2 et 3.

La culture de micro-algues s'effectue sur plusieurs jours et le milieu de culture contient environ 1g par litre de matière sèche de micro-algues.

La production de ces micro-algues se fait en condition naturelle d'éclairement et le traitement des micro-algues en vue d'en extraire les anti-oxydants s'effectue entre 12 et 18 heures et en phase exponentielle de croissance des micro-algues.

La culture soutirée du photobioréacteur est placée dans un bain-marie 4 porté entre 40 et 50°C, pendant 10 à 20 minutes en vue d'augmenter la perméabilité membranaire des micro-algues et par conséquent la teneur en anti-oxydants exocellulaires. Ce chauffage est réalisé sous agitation.

Comme indiqué en 6, la culture 1 est ensuite filtrée sur un filtre rotatif 7 avec un débit voisin de 200 l/h/m² afin de séparer les micro-algues du milieu liquide. Cette étape peut ainsi être effectuée sur une centrifugeuse continue entre 10 000 et 30 000 g avec un débit de 1 m³/h. Le filtrat clair 9 obtenu en 6 contient plus de la moitié des enzymes superoxydesdismutases. En particulier l'activité enzymatique mesurée dans le milieu de liquide est de 10 à 30 U/ml, la mesure étant effectuée par le NBT.

Les SOD sont directement en solution aqueuse et peuvent être concentrées ensuite, comme indiqué en 8, par ultrafiltration avec une membrane minérale de 10 000 daltons.

Le filtrat 9 ainsi concentré peut alors être purifié, comme indiqué en 10, en particulier par chromatographie échangeuse d'anions.

Les étapes de concentration 8 et de purification 10 sont effectuées à 4°C environ.

Le produit solide obtenu en 6 contient essentiellement les micro-algues. Ces dernières sont diluées, sous agitation, comme indiqué an 12, dans une solution de tampon phosphate (50 mM, Ph 7,8) introduite en 13 afin d'obtenir une concentration en micro-algues optimale pour l'étape de "broyage" symbolisée en 14. La concentration des micro-algues dans la suspension 15 obtenue en 12 est de 20 à 100 g/l de matière sèche (MS).

La suspension 15 est alors soumise à un cycle de pression-dépression dans un homogénéisateur ATV du type Manton-Gaulin fonctionnant à une pression de 2 à 5.10⁷ Pa. Cette étape est réalisée de préférence à 4°C. Elle a pour but d'éclater les micro-algues afin de rendre tout le contenu cellulaire accessible aux solvants.

A la suspension cellulaire de _Porphyridium_ _cruentum_ obtenue en 14, on ajoute une huile végétale ou minérale et par exemple une huile de soja ou un solvant type acétone ou méthanol pour extraire à la fois les anti-oxydants liposolubles tels que les tocophérols et les anti-oxydants hydrosolubles tels que la vitamine C et la SOD.

Cette addition de solvant est représentée en 16 et la flèche 17 indique l'amenée dans la suspension cellulaire du solvant. Cette addition de solvant se fait sous agitation et est effectuée à 4°C environ. La suspension cellulaire obtenue porte la référence 19.

Comme indiqué en 18, la suspension cellulaire 19 est ensuite centrifugée dans un extracteur centrifuge mono-étage équipé de vannes à piston du type VA 35-09-566. Le centrifugat obtenu est constitué en particulier d'une solution aqueuse contenant la SOD et la vitamine C et d'une solution lipidique contenant les tocophérols. La référence 21 correspond à la récupération de la solution aqueuse et la référence 23 à la récupération de la solution lipidique.

Ces solutions peuvent alors être concentrées, comme représenté en 8, de la mène façon que le milieu liquide obtenu après séparation en 6 des micro-algues.

Les résidus solides obtenus en 25 résultant de la centrifugation 18, contiennent des fragments de micro-algues.

Sur la figure 2, on a représenté un photobioréacteur de l'invention destine à la culture en "batch" de micro-algues ou de cyanobactéries productrices d'anti-oxydants.

Ce photobioréacteur clos 2 comporte un récepteur solaire 30 constitué de tubes parallèles 32 en matière plastique souple transparente telle que du polyéthylène dans lesquels circule le milieu de culture 1 contenant les micro-organismes et les éléments nutritifs nécessaires à leur croissance. Des collecteurs 34 et 36 en polypropylène permettent de connecter entre eux les tubes 32 et d'assurer le passage du liquide d'un tube à l'autre.

Ce récepteur solaire 30 est destiné à être placé à la surface d'une étendue d'eau en vue de sa régulation thermique.

Pour de plus amples détails sur la réalisation et le fonctionnement de ce capteur solaire on peut se référer au document FR-A-2 621 323 cité précédemment.

L'alimentation en $CO_2$ du milieu de culture, pendant un cycle ou "run", est assurée à l'aide d'un carbonateur 38 connecté à l'entrée du capteur solaire 30, via une conduite d'amenée 40; cette dernière est équipée d'une pompe 42 assurant la circulation du milieu de culture dans l'ensemble du photobioréacteur.

Le carbonateur 38 est du type à colonne et l'alimentation en $CO_2$ du milieu de culture via la conduite 40 est assurée par a base du carbonateur.

Le photobioréacteur comporte en outre une cuve 44 servant à la préparation, sous agitation, du milieu nourricier nécessaire à la croissance des micro-algues. Ce milieu nourricier est introduit en début de cycle dans le milieu liquide contenant les micro-algues, via une conduite d'amenée 46 montée en dérivation sur le tube d'amenée 40. Ce tube de dérivation 46 est équipé d'une vanne 47 permettant de contrôler l'injection du milieu nourricier.

Dans le récepteur solaire 30, la réaction de photosynthèse conduit à la formation d'oxygène. Des tubes de purge 48 montés en sortie du récepteur solaire 30, et plus précisément en sortie des collecteurs 34 et 36, permettent d'éliminer les poches de gaz qui se forment dans les tubes 32 du récepteur solaire. Ce gaz peut contenir jusqu'à 75% en volume d'oxygène dans des conditions de fort ensoleillement du récepteur solaire 30, oxygène provenant de la photosynthèse.

Cet oxygène gazeux est récolté dans un collecteur 50 puis est réinjecté dans le carbonateur 38 à l'aide d'une conduite 49. Le recyclage de l'oxygène gazeux se fait à la base du carbonateur 38.

Dans le carbonateur, l'oxygène est redissous dans le milieu de culture. Pour favoriser le passage de l'oxygène dans le milieu de culture, la partie supérieure du carbonateur est équipée d'une plaque déflectrice 51 de forme conique.

L'apport en $CO_2$ dans le carbonateur 38 est effectué en mélange avec de l'air, jusqu'à 80% en volume de $CO_2$ dans le mélange. Ce mélange gazeux est introduit, via une conduite d'alimentation 53, dans une cuve 52.

La sortie de cette cuve est équipée d'une conduite d'amenée 54 reliée à un tube plongeur central 56 du carbonateur 38, disposé selon l'axe de révolution de ce dernier. L'extrémité plongeante 58 du tube 56 est réalisée en verre ou inox fritté de porosité variable afin d'améliorer la dissolution à contre-courant du $CO_2$ dans le milieu liquide contenant les micro-algues. L'introduction du milieu de culture 1 dans le carbonateur s'effectue dans la partie supérieure de ce dernier grâce à une conduite d'amenée 60 montée en sortie du collecteur aval 36 du récepteur solaire.

Le mélange air plus $CO_2$ introduit dans le carbonateur 38 par le verre fritté 58 provoque une légère désorption de l'oxygène dissous dans le milieu de culture. Le mélange gazeux évacué au sommet du carbonateur est récupéré par une conduite d'évacuation 62 pour être réinjecté dans la cuve 52.

Le gaz de cette cuve 52 est comprimé grâce à un dispositif 64 du type à piston et est réinjecté dans la colonne de carbonation 38 via la conduite 54 puis le tube plongeur 56.

Ce gaz réinjecté à la base du carbonateur contient l'oxygène désorbé et le $CO_2$ qui ne s'est pas dissous dans la phase liquide.

La cuve 52 est munie d'un pressiostat 66 dont le niveau de pression est réglé entre 0,1 et $0,5.10^5$ Pa.

Ce système permet un enrichissement de la culture en oxygène dissous.

Dans les conditions de fort ensoleillement, le pourcentage d'oxygène dans la phase gazeuse est de 21% en volume en entrée de la conduite d'alimentation 53.

A la sortie du carbonateur 58, ou à l'entrée du récepteur solaire 30, c'est-à-dire dans le tube 40, le mélange gazeux contient 70% en volume d'oxygène. Ceci provient du fait que la culture arrivant en 60 dans le carbonateur est très riche en oxygène dissous provenant de la photosynthèse.

A la sortie du récepteur solaire, c'est-à-dire dans les tubes de purge 48, le mélange gazeux contient 75% en volume d'oxygène. Il y a donc un enrichissement de 70 à 75% en volume d'oxygène dans le récepteur solaire 30 par suite du confinement du milieu de culture.

La désoxygénation partielle ou désorption de l'oxygène au niveau du carbonateur abaisse la teneur en oxygène de la phase gazeuse de 75 à 70% dans ce dernier.

Dans ces conditions oxydantes, les micro-algues cultivées réagissent en synthétisant des anti-oxydants.

En particulier, la micro-algue Porphyridium cruentum réagit en produisant de la SOD à une moyenne de 10 à 20 U (NBT)/ml de culture, soit $5 \times 10^6$ à $20 \times 10^6$ U par $m^3$ et de la vitamine C à raison de 5 à 7 g/kg. En revanche, la micro-algue Haematococcus pluvialis réagit en produisant de la vitamine E à raison de 4 à 5 g/kg de micro-algues et du gamma-tocophérol à raison de 0,5 à 1 g/kg de micro-algues.

Le dispositif représenté sur la figure 2 comporte en outre une conduite de dérivation 68 équipée d'une vanne 70, montée sur la conduite de sortie 60. Cette conduite 68 débouche dans une cuve 70 destinée à récolter les micro-algues et le milieu de culture après un cycle de culture en vue d'en extraire les anti-oxydants.

Les conditions favorables de production d'anti-oxydants sont les conditions de forte activité photosynthétique. Aussi, on peut optimiser ces conditions à l'échelle d'un "run" ou cycle de culture de 10 à 20 jours. L'activité photosynthétique des micro-algues est maximale en début de "run", c'est-à-dire en phase exponentielle de croissance. Ceci est illustré par l'exemple ci-après.

Au cours d'un "run" effectué sur une culture de Porphiridium cruentum, les inventeurs ont noté les teneurs suivantes en SOD:

| | | |
|---|---|---|
| 5ème jour : | 25 U(NBT)/ml de culture (1,0 g MS/l de culture), soit 25 000 U/g de culture. | |
| 10ème jour: | 31 U (NBT)/ml de culture (1,3 g MS/l de culture), soit 24 000 U/g de culture. | |
| 17ème jour: | 27 U (NBT)/ml de culture (1,8 g MS/l de culture), soit 15 000 U/g de culture. | |
| 26ème jour: | 3 U (NBT)/ml de culture (2,0 g MS/l de culture). 1 500 U (NBT)/g de culture. | |

De cet exemple, il ressort clairement que la production maximale d'anti-oxydants s'effectue du 1er au 10ème jour d'un "run".

Des mesures similaires ont été effectuées au cours d'un "run" avec Heamatococcus pluvialis comme micro-algue. Les inventeurs ont noté les teneurs suivantes en tocophérols dans les micro-algues:

| | |
|---|---|
| 2ème jour : | 4 500 ppm d'alpha-tocophérol, 660 ppm de gamma-tocophérol. |
| 20ème jour: | 80 ppm d'alpha-tocophérol, 130 ppm de gamma-tocophérol. |

Selon l'invention les micro-organismes sont cultivés dans le photobioréacteur clos en condition naturelle d'éclairement, c'est-à-dire avec une alternance de jour et de nuit. L'assimilation de $CO_2$ par ces micro-organismes et par conséquent la production d'$O_2$ sont directement liées à l'intensité lumineuse. Elles sont donc optimales à la mi-journée.

Le schéma représenté sur la figure 3 fait apparaître l'évolution de la pression en $CO_2$ dissous et de la pression en oxygène dissous dans le milieu de culture au cours d'une journée. Les pressions de ces gaz sont données en partie par million. La courbe A donne les pressions de $CO_2$ et la courbe B les pressions d'$O_2$. La courbe C donne l'énergie solaire ES au cours de la journée ; elle est exprimée en kilojoules/$m^2$/s.

De ces courbes, il ressort clairement que les meilleurs conditions de production d'anti-oxydants se situent entre 12 et 18 heures. Par conséquent, la récolte des anti-oxydants se fera à cette période là.

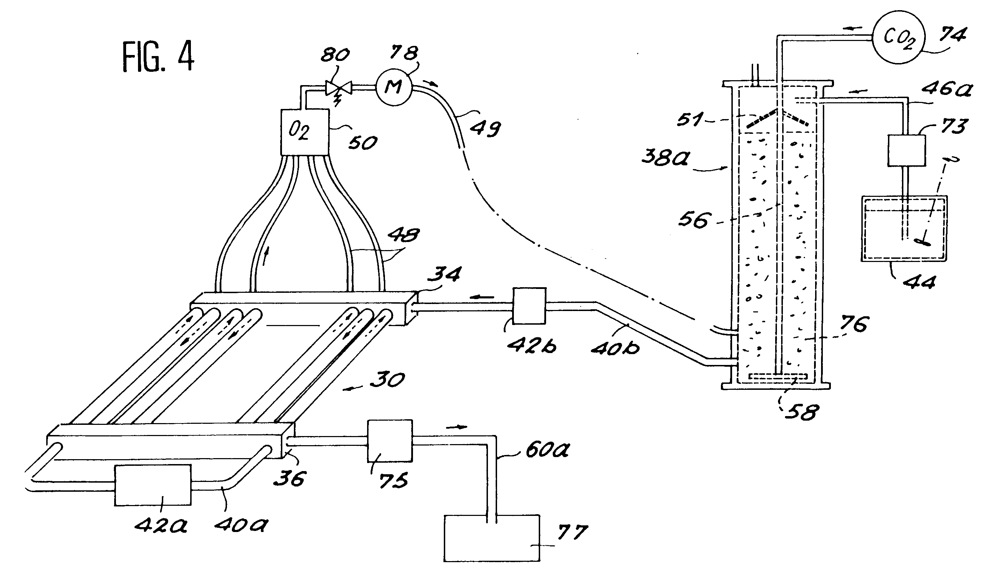

La culture des micro-algues et donc la production des anti-oxydants peut aussi se faire en continu à l'aide du photobioréacteur représenté sur la figure 4. Les éléments constitutifs de ce photobioréacteur identiques à ceux du photobioréacteur de la figure 2 portent les mêmes références.

Ce photobioréacteur comporte, comme sur la figure 2, un récepteur solaire tubulaire 30 équipé des collecteurs amont et aval 34 et 36. Une pompe 42a montée sur une conduite 40a mettant en communication l'entrée et la sortie du collecteur 34 fonctionne en continu. Elle assure la circulation en continu du milieu de culture contenant les micro-algues dans le récepteur solaire 30. L'ensemble récepteur solaire, conduite 40a et pompe 42a forme un ensemble clos, c'est-à-dire que les micro-organismes ne sortent pas de cet ensemble.

Le milieu nourricier préparé sous agitation dans la cuve 44 est introduit en continu, dans le cas présent, au sommet du carbonateur 38a grâce à une conduite d'amenée 46a équipée d'une pompe 73 fonctionnant en continu, assurant en outre le réglage du débit du milieu nourricier et donc le taux de renouvellement du milieu nourricier (1 par jour).

Le tube plongeur 56, équipé de son verre fritté 58 à son extrémité plongeante permet une alimentation en $CO_2$ à contre-courant du milieu nourricier dans le carbonateur 38a.

Afin d'améliorer la dissolution du gaz $CO_2$ injecté sous pression grâce au réservoir 74 monté à l'entrée du tube plongeur 56, la colonne du carbonateur est équipée d'un garnissage 76 du type anneau de Raschig ou selle de Bert.

Le milieu nourricier carbonaté est alors injecté dans le récepteur solaire 30 par une conduite d'amenée 40b reliant la base du carbonateur 38a au collecteur 34 du récepteur solaire 30. Cette conduite 40b est équipée d'une pompe 42b fonctionnant en continu et réglée au même débit que les pompes 73 et 75.

En sortie du récepteur solaire 30, une conduite 60a équipée d'une pompe 75 permet l'acheminement en continu dans une cuve 77 destinée à récolter le milieu de culture contenant les micro-organismes, en vue d'en extraire les anti-oxydants.

Comme précédemment dans le récepteur solaire 30, la photosynthèse provoque un enrichissement de la culture en oxygène dissous. Les poches de gaz formées renfermant de l'oxygène sont évacuées du récepteur 30 grâce aux tubes de purge 48 en direction du collecteur d'oxygène 50.

Ce collecteur 50 est muni d'un manomètre 78 pour mesurer la pression du gaz de purge enrichi en oxygène.

Lorsque la pression arrive entre 0,4 et $0,5.10^5$ Pa, une électrovanne 80 libère le gaz accumulé dans le collecteur 50. Ce gaz est alors réinjecté grâce à la conduite 49 à le base du carbonateur 38a de façon que l'oxygène gazeux résultant de la photosynthèse se dissolve dans la milieu de culture.

L'accumulation du gaz dans le récepteur solaire 30 engendre, comme précédemment, un enrichissement en oxygène dissous.

Pour augmenter la concentration en $O_2$ dissous dans le milieu liquide du photobioréacteur, il est souhaitable de le faire fonctionner en surpression. Dans l'exemple représenté, les tubes 32 du récepteur solaire ne résistent pas à une pression supérieure à $0,5.10^5$ Pa. Aussi, le déclenchement de l'électrovanne 80 est effectué entre 0,4 et $0,5.10^5$ Pa. La pression normale du gaz enrichi en oxygène se situe entre 0,1 et $0,2.10^5$ Pa.

Dans le photobioréacteur représenté sur la figure 2, le carbonateur 38 ne comporte pas de garnissage contrairement au carbonateur 38a de la figure 4 du fait que dans le fonctionnement en "batch", le milieu liquide contenant les micro-organismes traverse le carbonateur et ceux-ci adheraient au garnissage. Dans le carbonateur 38a, seuls les gaz et le milieu nourricier circulent.

Afin d'améliorer encore l'enrichissement en oxygène du milieu de culture, il est possible de remplacer le réservoir 74 de $CO_2$ sous pression du photobioréacteur de la figure 4 par le système de récupération 52-54-66 de l'oxygène désorbé dans le carbonateur, représenté sur la figure 2.

Avec Porphyridium cruentum cultivée et traitée selon l'invention, il est possible d'obtenir $5x10^6$ à $20x10^6$ unités NBT de SOD dans 1000 litres de culture, ce qui correspond à la production de 9 à 36 g de SOD par $m^3$ de culture. Dans ces conditions, des récepteurs solaires 30 repartis sur une surface d'eau d'un hectare, avec une exploitation de 200 jours/an, permettent la production de 90 à 360 kg de SOD.

**Revendications**

1. Procédé de production et d'extraction d'anti-oxydants, comportant les étapes suivantes :

   (a) - culture dans un photobioréacteur clos (2) de micro-organismes photosynthétiques en suspension dans un milieu liquide de culture (1), l'oxygène produit par les micro-organismes par photosynthèse étant collecté et réinjecté dans le milieu de culture, ces micro-organismes étant choisis dans le groupe constitué de micro-algues et des cyanobactéries,

   (b) - séparation (6) des micro-organismes du milieu de culture (1),

   (c) - mise en solution (12) des micro-organismes séparés en (b),

   (d) - "broyage" (14) des micro-organismes en solution (15),

   (e) - addition de solvant (16) à la solution obtenue en (d) pour solubiliser les anti-oxydants produits par les micro-organismes, puis

   (f) - séparation (18) des phases liquides et solides en présence.

2. Procédé selon la revendication 1, caractérisé en ce que l'étape (b) est réalisée pendant la phase exponentielle de croissance des micro-organismes.

3. Procédé selon la revendication 1 ou 2, caractérisé en ce que la culture des micro-organismes est effectuée en condition naturelle d'éclairement et en ce que l'étape (b) est effectuée dans l'après-midi.

4. Procédé selon l'une quelconque des revendications 1 à 3, caractérisé en ce qu'il comprend une étape de traitement (4) du milieu de culture (1) pour augmenter la production des anti-oxydants consistant en un chauffage ou une ionisation du milieu de culture.

5. Procédé selon l'une quelconque des revendications 1 à 4, caractérisé an ce que le solvant est un solvant organique ou de l'huile végétale ou minérale.

6. Procédé selon l'une quelconque des revendications 1 à 5, caractérisé en ce que les étapes (d), (e) et (f) sont effectuées à 4°C environ.

7. Procédé selon l'une quelconque des revendications 1 à 6, caractérisé en ce que les phases liquides obtenues en (f) sont concentrées (8) puis purifiées (10).

8. Procédé selon l'une quelconque des revendications 1 à 7, caractérisé en ce que le milieu de culture séparé en (b) est concentré (8) puis purifié (10).

9. Procédé selon l'une quelconque des revendications 1 à 8, caractérisé en ce que la solution (15) de l'étape (c) contient de 20 à 100 g/l de micro-organismes secs.

10. Procédé selon l'une quelconque des revendications 1 à 9, caractérisé en ce que l'oxygène produit par photosynthèse est réinjecté sous pression dans le milieu de culture (1).

11. Procédé selon l'une quelconque des revendications 1 à 10, caractérisé en ce que les micro-organismes sont choisis parmi Porphyridium cruentum et Haematococcus pluvialis.

12. Photobioréacteur clos pour la production d'anti-oxydants à partir d'une culture de micro-organismes photosynthétiques en suspension dans un milieu liquide, ces micro-organismes étant choisis dans le groupe constitué des micro-algues et des cyanobactéries, ce photobioréacteur comportant :

    (A) - un récepteur solaire (30) transparent à la lumière solaire dans lequel circule le milieu liquide (1),

    (B) - un carbonateur à colonne (38, 38a) raccordé à l'entrée (34) du récepteur solaire pour charger le milieu liquide en $CO_2$ nécessaire à la photosynthèse,

    (C) - un collecteur (50) de l'oxygène produit par les micro-organismes par photosynthèse, et

    (D) - des moyens (49, 52, 54, 66, 78, 80) pour réinjecter l'oxygène collecté au niveau du carbonateur.

13. Photobioréacteur selon la revendication 12, caractérisé en ce que le récepteur solaire (30) est constitué de tubes parallèles (32) dans lesquels circule le milieu liquide, des collecteurs d'extrémité (34, 36) assurant la mise en communication de ces tubes.

14. Photobioréacteur selon la revendication 12 ou 13, caractérisé en ce que le carbonateur (38, 38a) comporte dans sa partie supérieure une plaque (51) de dispersion du milieu liquide.

15. Photobioréacteur selon l'une quelconque des revendications 12 à 14, destiné à la culture en continu des micro-organismes, caractérisé en ce que le carbonateur (38a) comporte un garnissage (76) pour favoriser l'introduction du

$CO_2$ et de l'$O_2$ dans le milieu liquide.

16. Photobioréacteur selon l'une quelconque des revendications 12 à 15, caractérisé en ce que le carbonateur (38, 38a) comporte un tube plongeur central (56) logé dans la colonne, terminé par un verre ou inox fritté (58) de porosité variable pour alimenter le carbonateur en $CO_2$ par le fond et une conduite d'amenée (46a, 60) du milieu liquide (1) dans la partie supérieure du carbonateur de sorte que le milieu liquide et le gaz contenant le $CO_2$ circulent à contre-courant dans le carbonateur.

17. Photobioréacteur selon le revendication 16, caractérisé en ce qu'il comprend en outre, une cuve d'admission (52) de $CO_2$, équipée d'une conduite d'alimentation (53) en $CO_2$, une conduite (34) pour amener le gaz de la cuve à la base du carbonateur (38) à l'aide du tube plongeur (56), une conduite d'évacuation des gaz (64) montée au sommet du carbonateur pour réinjecter les gaz évacués dans la cuve, des moyens (64) pour comprimer les gaz dans la cuve et des moyens (66) pour régler la pression des gaz introduits dans le tube plongeur.

18. Photobioréacteur selon l'une quelconque des revendications 12 à 17, caractérisé en ce qu'il comprend des moyens (78, 80) pour contrôler la pression du gaz enrichi en oxygène réinjecté dans le milieu de culture (1).

**Claims**

1. Process for the production and extraction of antioxidants, comprising the following stages:
   (a) culturing in a closed photobioreactor (2) of photosynthetic microorganisms suspended in a liquid culture medium (1), the oxygen produced by the microorganisms by photosynthesis being collected and reinjected into the culture medium, said microorganisms being chosen from the group constituted by microalgae and cyanobacteria,
   (b) separation (6) of the microorganisms from the culture medium,
   (c) dissolving (12) the microorganisms separated in (b),
   (d) "crushing" (14) the dissolved microorganisms,
   (e) addition of solvent (16) to the solution obtained in (d) in order to solubilize the antioxidants produced by the microorganisms and then
   (f) separation (18) of the liquid and solid phases present.

2. Process according to claim 1, characterized in that stage (b) is performed during an exponential growth phase of the microorganisms.

3. Process according to claim 1 or 2, characterized in that the microorganisms are cultured under natural lighting conditions and in that stage (b) is performed in the afternoon.

4. Process according to any one of the claims 1 to 3, characterized in that it comprises a stage (4) of treating the culture medium (1) in order to increase the production of antioxidants consisting of a heating or ionization of the culture medium.

5. Process according to any one of the claims 1 to 4, characterized in that the solvent is an organic solvent or vegetable or mineral oil.

6. Process according to any one of the claims 1 to 5, characterized in that stages (d), (a) and (f) are performed at approximately 4°C.

7. Process according to any one of the claims 1 to 6, characterized in that the liquid phases obtained in (f) are concentrated (8) and then purified (10).

8. Process according to any one of the claims 1 to 7, characterized in that the culture medium separated in (b) is concentrated (8) and then purified (10).

9. Process according to any one of the claims 1 to 8, characterized in that the solution (15) of stage (c) contains 20 to 100 g/l of dry microorganisms.

10. Process according to any one of the claims 1 to 9, characterized in that the oxygen produced by photosynthesis is reinjected under pressure into the culture medium (1).

11. Process according to any one of the claims 1 to 10, characterized in that the microorganisms are chosen from among Porphyridium cruentum and Haematococcus pluvialis.

12. Closed photobioreactor for the production of antioxidants from a culture of photosynthetic microorganisms suspended in a liquid medium, said microorganisms being chosen from the group constituted by microalgae and cyanobacteria, said photobioreactor comprising:
    (A) a solar receiver (30) transparent to sunlight in which circulates the liquid medium (1),

(B) a column carbonator (38,38a) connected to the intake (34) of the solar receiver for supplying the liquid medium with the $CO_2$ necessary for photosynthesis,

(C) a collector (50) of the oxygen produced by the microorganisms by photosynthesis and

(D) means (49,52,54,66,78,80) for reinjecting the collected oxygen with respect to the carbonator.

13. Photobioreactor according to claim 12, characterized in that the solar receiver (30) is constituted by parallel tubes (32) in which the liquid medium circulates, the end collectors (34,36) linking the said tubes.

14. Photobioreactor according to claim 12 or 13, characterized in that in its upper part the carbonator (38,38a) has a liquid medium dispersion plate (51).

15. Photobioreactor according to any one of the claims 12 to 14, for the continuous culturing of microorganisms, characterized in that the carbonator (38a) has a lining (76) for aiding the introduction of $CO_2$ and $O_2$ into the liquid medium.

16. Photobioreactor according to any one of the claims 12 to 15, characterized in that the carbonator (38,38a) has a central immersion tube (56) located in the column and terminated by a variable porosity fritted or sintered stainless material or glass member (58) for supplying the carbonator with $CO_2$ through its bottom and a supply pipe (46a,60) for the liquid medium (1) to the upper part of the carbonator, in such a way that the liquid medium and the $CO_2$-containing gas flow in counter-current manner in the carbonator.

17. Photobioreactor according to claim 16, characterized in that it also comprises a $CO_2$ admission vessel (52) equipped with a $CO_2$ supply pipe (53), a pipe (34) for supplying gas from the vessel to the bottom of the carbonator (38) with the aid of the immersion tube (56), a gas discharge pipe (64) mounted at the top of the carbonator in order to reinject the discharged gases into the vessel, means (64) for compressing the gases in the vessel and means (66) for regulating the pressure of the gases introduced into the immersion tube.

18. Photobioreactor according to any one of the claims 12 to 17, characterized in that it comprises means (78,80) for checking or controlling the pressure of the oxygen-enriched gas reinjected into the culture medium (1).

**Patentansprüche**

1. Verfahren zur Herstellung und Extraktion von Antioxidantien, das aus folgenden Arbeitsschritten besteht:

(a) - Züchtung von photosynthetischen Mikroorganismen in einem abgeschlossenen Photobioreaktor (2) in Suspension in einem flüssigen Kulturmedium (1), wobei der von den Mikroorganismen durch Photosynthese erzeugte Sauerstoff gesammelt und in das Kulturmedium zurückgeführt wird und die Mikroorganismen aus der Gruppe der Mikroalgen oder Zyanobakterien ausgewählt werden,

(b) - Trennung (6) der Mikroorganismen von dem Kulturmedium (1),

(c) - Lösung (12) der in Arbeitsschritt (b) abgeschiedenen Mikroorganismen,

(d) - "Zerkleinern" (14) der Mikroorganismen in der Lösung (15),

(e) - Beimengung eines Lösungsmittels (16) zu der in Arbeitsschritt (d) gewonnenen Lösung, um die durch die Mikroorganismen erzeugten Antioxidantien löslich zu machen, danach

(f) - Trennung (18) der vorhandenen flüssigen von der festen Phase.

2. Verfahren nach Anspruch 1, dadurch gekennzeichnet, daß der Arbeitsschritt (b) während der exponentiellen Phase des Mikroorganismenwachstums durchgeführt wird.

3. Verfahren nach Anspruch 1 oder 2, dadurch gekennzeichnet, daß die Züchtung der Mikroorganismen unter natürlichen Lichtverhältnissen erfolgt und daß der Arbeitsschritt (b) nachmittags durchgeführt wird.

4. Verfahren nach einem der Ansprüche 1 bis 3, dadurch gekennzeichnet, daß es einen Arbeitsschritt (4) beinhaltet, bei dem das Kulturmedium (1) behandelt wird, um die Produktion von Antioxidantien zu steigern, wobei dieser Arbeitsschritt darin besteht, daß das Kulturmedium erhitzt oder ionisiert wird.

5. Verfahren nach einem der Ansprüche 1 bis 4, dadurch gekennzeichnet, daß das Lösungsmittel ein organisches Lösungsmittel, ein pflanzliches Öl oder ein mineralisches Öl ist.

6. Verfahren nach einem der Ansprüche 1 bis 5, dadurch gekennzeichnet, daß die Arbeitsschritte (d), (e) und (f) bei etwa 4 °C durchgeführt werden.

7. Verfahren nach einem der Ansprüche 1 bis 6, dadurch gekennzeichnet, daß die in Arbeitsschritt (f) gewonnenen flüssigen Phasen konzentriert (8) and anschließend gereinigt (10) werden.

8. Verfahren nach einem der Ansprüche 1 bis 7, dadurch gekennzeichnet, daß dar in Arbeitsschritt (b) abgesonderte Kulturmedium erst konzentriert (8) und dann gereinigt (10) wird.

9. Verfahren nach einem der Ansprüche 1 bis 8, dadurch gekennzeichnet, daß die Lösung (15) aus Arbeitsschritt (c) 20 bis 100 g/l getrocknete Mikroorganismen enthält.

10. Verfahren nach einem der Ansprüche 1 bis 9, dadurch gekennzeichnet, daß der durch die Photosynthese erzeugte Sauerstoff unter Druck in das Kulturmedium (1) wieder eingespritzt wird.

11. Verfahren nach einem der Ansprüche 1 bis 10, dadurch gekennzeichnet, daß die Mikroorganismen aus den Gruppen Porphyridus cruentus und Haematococcus pluvialis ausgewählt werden.

12. Abgeschlossener Photobioreaktor zur Herstellung von Antioxidantien ausgehend von einer photosynthetischen Mikroorganismenkultur in Suspension in einem flüssigen Medium, wobei diese Mikroorganismen aus der Gruppe der Mikroalgen und Zyanobakterien stammen und dieser Photobioreaktor aus folgenden Teilen besteht:

(A) - einem für das Sonnenlicht durchlässigen Sonnenkollektor (30), in dem das flüssige Medium (1) zirkuliert,

(B) - einem Säulenkarbonateur (38, 38a), der an den Zufluß (34) des Sonnenkollektors angeschlossen wird, damit das flüssige Medium mit dem für die Photosynthese erforderlichen $CO_2$ angereichert werden kann,

(C) - einem Sauerstoffkollektor (50), der den von den Mikroorganismen durch Photosynthese erzeugten Sauerstoff auffängt, und

(D) - Vorrichtungen (49, 52, 54, 66, 78, 80) zum Wiedereinspritzen des aufgefangenen Sauerstoffs am Karbonateur.

13. Photobioreaktor nach Anspruch 12, dadurch gekennzeichnet, daß der Sonnenkollektor (30) aus parallel zueinander verlaufenden Rohren (32) besteht, in denen das flüssige Medium zirkuliert, wobei die an den Enden befindlichen Kollektoren (34, 36) die Verbindung mit diesen Rohren herstellen.

14. Photobioreaktor nach Anspruch 12 oder 13, dadurch gekennzeichnet, daß der Karbonateur (38, 38a) in dessen oberen Teil eine Scheibe (51) zur Dispergierung des flüssigen Mediums enthält.

15. Photobioreaktor nach einem der Ansprüche 12 bis 14, der für die Züchtung von Mikroorganismen im Dauerbetrieb ausgelegt ist, dadurch gekennzeichnet, daß der Karbonateur (38a) eine Bestückung (76) enthält, die dazu dient, die Einleitung des $CO_2$ und des $O_2$ in das flüssige Medium zu begünstigen.

16. Photobioreaktor nach einem der Ansprüche 12 bis 15, dadurch gekennzeichnet, daß der Karbonateur (38, 38a) ein zentrales in der Säule untergebrachtes Tauchrohr (56), das von einem Teil (58) aus gesintertem Glas oder Edelstahl mit variabler Porosität verschlossen ist, das dazu dient, den Karbonateur von unten her mit $CO_2$ zu versorgen, sowie eine Zufuhrleitung (46a, 60) besitzt, die der Beförderung des flüssigen Mediums (1) in den oberen Teil des Karbonateurs dient, was auf die Weise erfolgt, daß das flüssige Medium und das das $CO_2$ enthaltende Gas in dem Karbonateur nach dem Gegenstromprinzip zirkulieren.

17. Photobioreaktor nach Anspruch 16, dadurch gekennzeichnet, daß dieser unter anderem einen $CO_2$-Aufnahmebehälter (52) enthält, der mit einer $CO_2$-Speiseleitung (53), einer Leitung (34) zur Zufuhr des Gases aus dem Behälter in den unteren Bereich des Karbonateurs (38) mit Hilfe des Tauchrohrs (56), einem Gasableitungsrohr (64), das im obersten Bereich des Karbonateurs montiert ist, um das abgeführte Gas in den Behälter wieder einzuspeisen, mit Vorrichtungen (64) zur Komprimierung des Gases in dem Behälter sowie mit Vorrichtungen (66) zur Regelung des Druckes des in das Tauchrohr eingeleiteten Gases versehen ist.

18. Photobioreaktor nach einem der Ansprüche 12 bis 17, dadurch gekennzeichnet, daß dieser Vorrichtungen (78, 80) zur Regelung des Druckes des Gases enthält, das mit dem in das Kulturmedium (1) wieder eingespritzten Sauerstoff angereichert wurde.

CO2
O2

ANTI-OX

SOLV.

FIG.1

FIG. 2

FIG. 3

FIG. 4